(19) Europäisches Patentamt
European Patent Office
Office européen des brevets

(11) Veröffentlichungsnummer: **0 602 427 A1**

(12) **EUROPÄISCHE PATENTANMELDUNG**

(21) Anmeldenummer: **93118990.6**

(51) Int. Cl.[5]: **C07D 401/12**, A01N 47/36

(22) Anmeldetag: **25.11.93**

(30) Priorität: **08.12.92 DE 4241303**

(43) Veröffentlichungstag der Anmeldung: **22.06.94 Patentblatt 94/25**

(84) Benannte Vertragsstaaten: **BE CH DE DK ES FR GB IT LI NL**

(71) Anmelder: **BAYER AG**

**D-51368 Leverkusen(DE)**

(72) Erfinder: **Müller, Klaus-Helmut, Dr.**
**Solfstrasse 19**
**D-40593 Düsseldorf(DE)**
Erfinder: **Santel, Hans-Joachim, Dr.**
**Grünstrasse 9a**
**D-51371 Leverkusen(DE)**
Erfinder: **Dollinger, Markus, Dr.**
**Hüschelrath 7**
**D-42799 Leichlingen(DE)**

(54) **Substituierte chinolylsulfonylharnstoffe.**

(57) Die Erfindung betrifft neue substituierte Chinolylsulfonylharnstoffe der Formel (I),

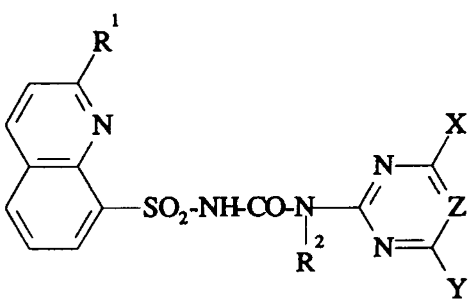

(I)

in welcher

- $R^1$ für gegebenenfalls durch Halogen substituiertes $C_1$-$C_6$-Alkyl steht,
- $R^2$ für Wasserstoff oder für gegebenenfalls durch Halogen, Cyano, Carboxy, $C_1$-$C_4$-Alkoxy oder $C_1$-$C_4$-Alkoxy-carbonyl substituiertes $C_1$-$C_6$-Alkyl, für jeweils gegebenenfalls durch Halogen substituiertes $C_3$-$C_6$-Alkenyl oder $C_3$-$C_6$-Alkinyl, oder für gegebenenfalls durch Halogen, Cyano, $C_1$-$C_4$-Alkyl, $C_1$-$C_4$-Alkoxy oder $C_1$-$C_4$-Alkoxy-carbonyl substituiertes Phenyl-$C_1$-$C_2$-Alkyl steht,
- X für Wasserstoff, Halogen, Cyclopropyl oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_3$-alkyl)-amino steht,
- Y für Wasserstoff, Halogen oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_3$-alkyl)-amino steht und
- Z für Stickstoff oder die Gruppierung C-$R^3$ steht,
  worin
- $R^3$ für Wasserstoff, Halogen, $C_1$-$C_4$-Alkyl oder $C_1$-$C_4$-Alkoxy steht,

sowie Salze von Verbindungen der Formel (I), wobei die Verbindung N-(4,6-Dimethy-s-triazin-2-yl)-N'-(2-methyl-chinolin-8-yl-sulfonyl)-harnstoff ausgenommen ist,
sowie Verfahren zur Herstellung der neuen Verbindungen und deren Verwendung als Herbizide.

EP 0 602 427 A1

Die Erfindung betrifft neue substituierte Chinolylsulfonylharnstoffe, Verfahren zu ihrer Herstellung und ihre Verwendung als Herbizide.

Es ist bekannt, daß bestimmte Chinolylsulfonylharnstoffe herbizide Eigenschaften aufweisen (vgl. EP-A 51465/US-P 4369320/US-P4453970). Die vorbekannten Chinolylsulfonylharnstoffe haben jedoch keine nennenswerte technische Bedeutung erlangt.

Es wurden nun neue substituierte Chinolylsulfonylharnstoffe der allgemeinen Formel (I)

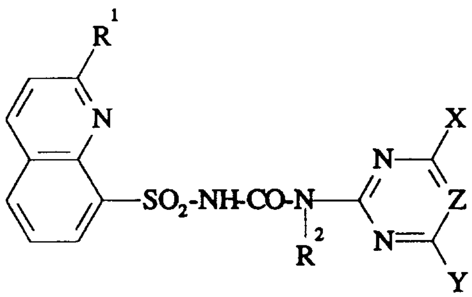

(I)

in welcher

$R^1$ für gegebenenfalls durch Halogen substituiertes $C_1$-$C_6$-Alkyl steht,

$R^2$ für Wasserstoff oder für gegebenenfalls durch Halogen, Cyano, Carboxy, $C_1$-$C_4$-Alkoxy oder $C_1$-$C_4$-Alkoxy-carbonyl substituiertes $C_1$-$C_6$-Alkyl, für jeweils gegebenenfalls durch Halogen substituiertes $C_3$-$C_6$-Alkenyl oder $C_3$-$C_6$-Alkinyl, oder für gegebenenfalls durch Halogen, Cyano, $C_1$-$C_4$-Alkyl, $C_1$-$C_4$-Alkoxy oder $C_1$-$C_4$-Alkoxy-carbonyl substituiertes Phenyl-$C_1$-$C_2$-Alkyl steht,

X für Wasserstoff, Halogen, Cyclopropyl oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_3$-alkyl)-amino steht,

Y für Wasserstoff, Halogen oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_3$-alkyl)-amino steht und

Z für Stickstoff oder die Gruppierung C-$R^3$ steht,
worin

$R^3$ für Wasserstoff, Halogen, $C_1$-$C_4$-Alkyl oder $C_1$-$C_4$-Alkoxy steht,

sowie Salze von Verbindungen der Formel (I) gefunden, wobei die vorbekannte Verbindung N-(4,6-Dimethyl-s-triazin-2-yl)-N'-(2-methyl-chinolin-8-yl-sulfonyl)-harnstoff (vgl. US-P 4369320/US-P 4453970) ausgenommen ist.

Man erhält die neuen substituierten Chinolylsulfonylharnstoffe der allgemeinen Formel (I) und deren Salze, wenn man

(a) substituierte Chinolinsulfonsäureamide der allgemeinen Formel (II)

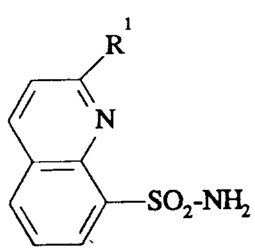

(II)

in welcher

$R^1$ die oben angegebene Bedeutung hat,

mit Urethanen (Carbamaten) der allgemeinen Formel (III)

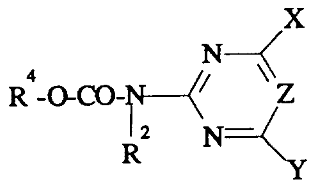

(III)

in welcher

$R^2$, X, Y und Z die oben angegebene Bedeutung haben und

$R^4$ für $C_1$-$C_4$-Alkyl, Benzyl oder Phenyl steht,

gegebenenfalls in Gegenwart eines Säureakzeptors und gegebenenfalls in Gegenwart eines Verdünnungsmittels umsetzt, oder wenn man

(b) substituierte Chinolinsulfonsäureamide der allgemeinen Formel (II)

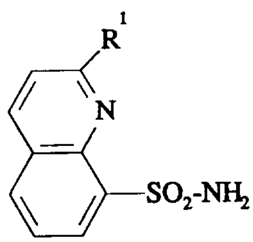

(II)

in welcher

$R^1$ die oben angegebene Bedeutung hat,

in einer ersten Reaktionsstufe mit Chlorameisensäureestern der allgemeinen Formel (IV)

$Cl\text{-}CO\text{-}O\text{-}R^4$ (IV)

in welcher

$R^4$ die oben angegebene Bedeutung hat,

gegebenenfalls in Gegenwart eines Säureakzeptors und gegebenenfalls in Gegenwart eines Verdünnungsmittels umsetzt, und die hierbei gebildeten Chinolylsulfonylurethane der allgemeinen Formel (V)

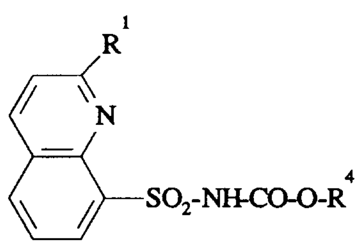

(V)

in welcher

$R^1$ und $R^4$ die oben angegebene Bedeutung haben,

in einer zweiten Reaktionsstufe mit Aminoazinen der allgemeinen Formel (VI)

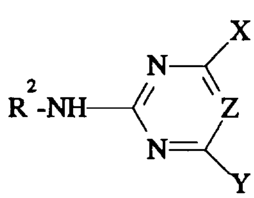

(VI)

in welcher

$R^2$, X, Y und Z die oben angegebene Bedeutung haben,

gegebenenfalls nach Zugabe einer Säure umsetzt,

und gegebenenfalls die nach Verfahren (a) oder (b) erhaltenen substituierten Chinolylsulfonylharnstoffe nach üblichen Methoden in Salze überführt.

Eine weitere mögliche Herstellungsmethode für die erfindungsgemäßen Verbindungen der Formel (I) ist nachstehend skizziert, wobei $R^1$, $R^2$, X, Y und Z die oben angegebene Bedeutung haben:

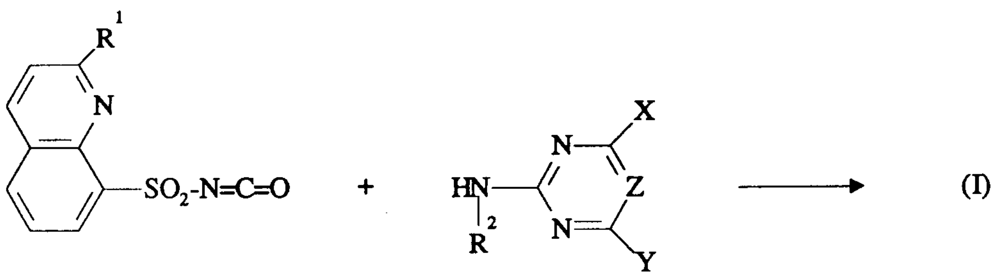

Die neuen substituierten Chinolylsulfonylharnstoffe der allgemeinen Formel (I) zeichnen sich durch starke herbizide Wirksamkeit aus.

Gegenstand der Erfindung sind vorzugsweise Verbindungen der Formel (I), in welcher

$R^1$ für gegebenenfalls durch Fluor und/oder Chlor substituiertes $C_1$-$C_4$-Alkyl steht,

$R^2$ für Wasserstoff oder für gegebenenfalls durch Fluor, Chlor, Cyano, Carboxy, Methoxy, Ethoxy, Methoxycarbonyl oder Ethoxycarbonyl substituiertes $C_1$-$C_4$-Alkyl, für jeweils gegebenenfalls durch Fluor, Chlor und/oder Brom substituiertes $C_3$-$C_4$-Alkenyl oder $C_3$-$C_4$-Alkinyl steht, oder für gegebenenfalls durch Fluor, Chlor, Brom, Cyano, Methyl, Ethyl, Methoxy, Ethoxy, Methoxycarbonyl oder Ethoxycarbonyl substituiertes Benzyl steht,

X für Wasserstoff, Fluor, Chlor, Brom, Cyclopropyl oder für jeweils gegebenenfalls durch Fluor, Chlor, Methoxy oder Ethoxy substituiertes $C_1$-$C_3$-Alkyl, $C_1$-$C_3$-Alkoxy, $C_1$-$C_3$-Alkylthio, $C_1$-$C_3$-Alkylamino oder Di-($C_1$-$C_2$-alkyl)-amino steht,

Y für Wasserstoff, Fluor, Chlor, Brom, oder für jeweils gegebenenfalls durch Fluor, Chlor, Methoxy oder Ethoxy substituiertes $C_1$-$C_3$-Alkyl, $C_1$-$C_3$-Alkoxy, $C_1$-$C_3$-Alkylthio, $C_1$-$C_3$-Alkylamino oder Di-($C_1$-$C_2$-alkyl)-amino steht und

Z für Stickstoff oder die Gruppierung C-$R^3$ steht, worin

$R^3$ für Wasserstoff, Fluor, Chlor, Brom, Methyl, Ethyl, Methoxy oder Ethoxy steht,

mit Ausnahme der vorbekannten Verbindung N-(4,6-Dimethyl-s-triazin-2-yl)-N'-(2-methyl-chinolin-8-yl-sulfonyl)-harnstoff.

Gegenstand der Erfindung sind weiter vorzugsweise Natrium-, Kalium-, Calcium-, Magnesium-, Ammonium-, $C_1$-$C_4$-Alkyl-ammonium-, Di-($C_1$-$C_4$-alkyl)-ammonium-, Tri-($C_1$-$C_4$-alkyl)-ammonium-, $C_5$- oder $C_6$-Cycloalkyl-ammonium- und Di-($C_1$-$C_2$-alkyl)-benzyl-ammonium-Salze von Verbindungen der Formel (I), in welcher $R^1$, $R^2$, X, Y und Z die oben vorzugsweise angegebenen Bedeutungen haben.

Gegenstand der Erfindung sind insbesondere Verbindungen der Formel (I), in welcher

$R^1$ für Methyl, Ethyl, Propyl oder Isopropyl steht,

$R^2$ für Wasserstoff oder Methyl steht,

X für Wasserstoff, Chlor, Cyclopropyl, Methyl, Ethyl, Trifluormethyl, Methoxymethyl, Methoxy, Ethoxy, Difluormethoxy, Methylthio, Ethylthio, Methylamino, Ethylamino oder Dimethylamino steht,

Y für Wasserstoff, Chlor, Trifluormethyl, Methoxy, Ethoxy, Difluormethoxy, Methylthio, Ethylthio, Methylamino, Ethylamino oder Dimethylamino steht und

Z für Stickstoff oder die Gruppierung C-$R^3$ steht, worin

$R^3$ für Wasserstoff steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- bzw. Zwischenprodukte. Diese Restedefinitionen können untereinander, also auch zwischen den angegebenen bevorzugten Bereichen beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Beispiele für die erfindungsgemäßen Verbindungen der Formel (I) sind in der nachstehenden Tabelle I aufgeführt.

Tabelle 1: Beispiele für die Verbindungen der Formel (I)

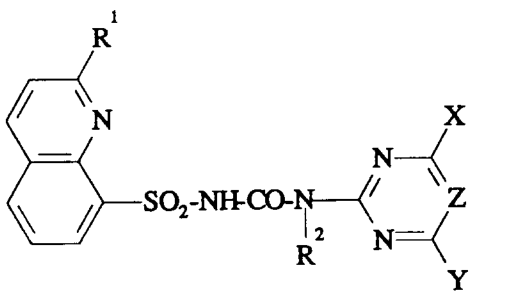

(I)

| $R^1$ | $R^2$ | X | Y | Z |
|---|---|---|---|---|
| $CH_3$ | H | $CH_3$ | $OCH_3$ | N |
| $CH_3$ | $CH_3$ | $CH_3$ | $OCH_3$ | N |
| $CH_3$ | H | $OCH_3$ | $OCH_3$ | N |
| $CH_3$ | $CH_3$ | $OCH_3$ | $OCH_3$ | N |
| $CH_3$ | H | $CF_3$ | $OCH_3$ | N |
| $CH_3$ | H | $SCH_3$ | $SCH_3$ | N |
| $CH_3$ | H | $NHCH_3$ | $OCH_3$ | N |
| $CH_3$ | H | $NHC_2H_5$ | $OCH_3$ | N |
| $CH_3$ | H | $NHCH_3$ | $OC_2H_5$ | N |
| $CH_3$ | H | $OCH_3$ | $N(CH_3)_2$ | N |
| $CH_3$ | H | $CH_3$ | $N(CH_3)_2$ | N |
| $CH_3$ | H | $CH_3$ | $OCH_3$ | CH |

Tabelle 1 - Fortsetzung

| $R^1$ | $R^2$ | X | Y | Z |
|---|---|---|---|---|
| $CH_3$ | H | $OCH_3$ | $OCH_3$ | CH |
| $CH_3$ | H | $CF_3$ | $OCH_3$ | CH |
| $CH_3$ | H | $OCH_3$ | Cl | CH |
| $CH_3$ | H | $OCHF_2$ | $OCHF_2$ | CH |
| $C_2H_5$ | H | $CH_3$ | $OCH_3$ | N |
| $C_2H_5$ | $CH_3$ | $CH_3$ | $OCH_3$ | N |
| $C_2H_5$ | H | $OCH_3$ | $OCH_3$ | N |
| $C_2H_5$ | $CH_3$ | $OCH_3$ | $OCH_3$ | N |
| $C_2H_5$ | $CH_3$ | $OC_2H_5$ | $OC_2H_5$ | N |
| $C_2H_5$ | H | $CH_3$ | $OCH_3$ | CH |
| $C_2H_5$ | H | $OCH_3$ | $OCH_3$ | CH |
| $C_2H_5$ | H | $CF_3$ | $OCH_3$ | CH |
| $C_2H_5$ | H | Cl | $OCH_3$ | CH |
| $C_2H_5$ | H | $OCHF_2$ | $OCHF_2$ | CH |
| $CH_3$ | H | 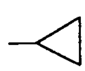 | $OCH_3$ | N |
| $CH_3$ | $CH_3$ | $SCH_3$ | $CH_3$ | N |
| $CH_3$ | H | 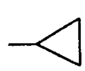 | $CH_3$ | N |

Tabelle 1 - Fortsetzung

| $R^1$ | $R^2$ | X | Y | Z |
|---|---|---|---|---|
| $CH_3$ | H | $OC_2H_5$ | $CF_3$ | CH |
| $CH_3$ | H | 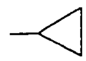 | $N(CH_3)_2$ | N |
| $CH_3$ | H | $OC_2H_5$ | $OC_2H_5$ | CH |
| $CH_3$ | H | $OC_2H_5$ | $OC_2H_5$ | N |
| $C_2H_5$ | H | 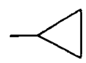 | $OCH_3$ | N |
| $CH_3$ | H | $OCH_2CF_3$ | $N(CH_3)_2$ | N |
| $CH_3$ | H | $OC_2H_5$ | $N(CH_3)_2$ | N |
| $CH_3$ | H | $OC_2H_5$ | Cl | CH |
| $CH_3$ | H | $OCHF_2$ | $OCH_3$ | CH |
| $CH_3$ | H | $OCHF_2$ | Cl | CH |
| $CH_3$ | H | $CH_3$ | Cl | CH |
| $CH_3$ | H | $CF_3$ | $CH_3$ | CH |
| $CH_3$ | H | $CF_3$ | $OCHF_2$ | CH |
| n-$C_3H_7$ | H | $OCH_3$ | $OCH_3$ | CH |
| n-$C_3H_7$ | H | $OCH_3$ | $OCH_3$ | N |
| n-$C_3H_7$ | H | $CH_3$ | $OCH_3$ | N |
| i-$C_3H_7$ | H | $OCH_3$ | $OCH_3$ | CH |

Tabelle 1 - Fortsetzung

| $R^1$ | $R^2$ | X | Y | Z |
|---|---|---|---|---|
| $i\text{-}C_3H_7$ | H | $OCH_3$ | $OCH_3$ | N |
| $i\text{-}C_3H_7$ | H | $OCH_3$ | $CH_3$ | N |

Verwendet man beispielsweise 2-Ethyl-chinolin-8-sulfonamid und N-(4,6-Dimethoxy-1,3,5-triazin-2-yl)-carbamidsäure-methylester als Ausgangsstoffe, so kann der Reaktionsablauf beim erfindungsgemäßen Verfahren (a) durch das folgende Formelschema skizziert werden:

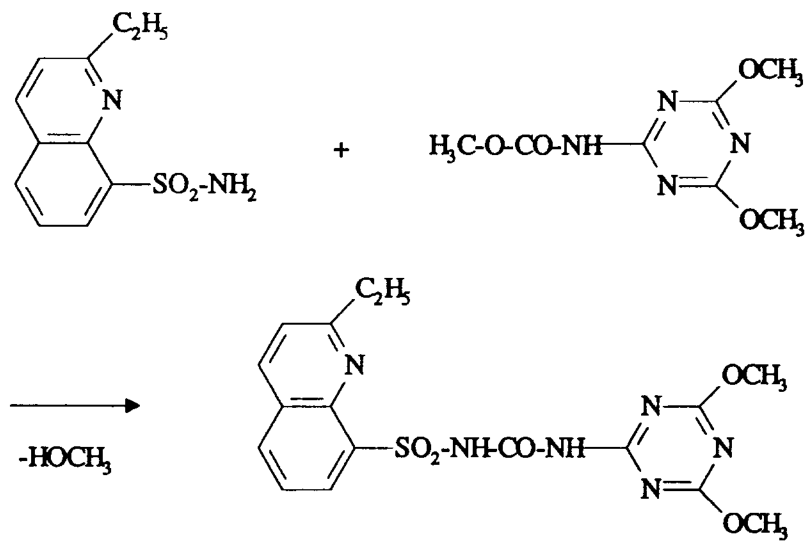


Verwendet man beispielsweise 2-Methyl-chinolin-8-sulfonamid und Chlorameisensäure-phenylester in der ersten Reaktionsstufe und 2-Amino-4,6-(bis-difluormethoxy)-pyrimidinin der zweiten Reaktionsstufe als Ausgangsstoffe, so kann der Reaktionsablauf beim erfindungsgemäßen Verfahren (b) durch das folgende Formelschema skizziert werden:

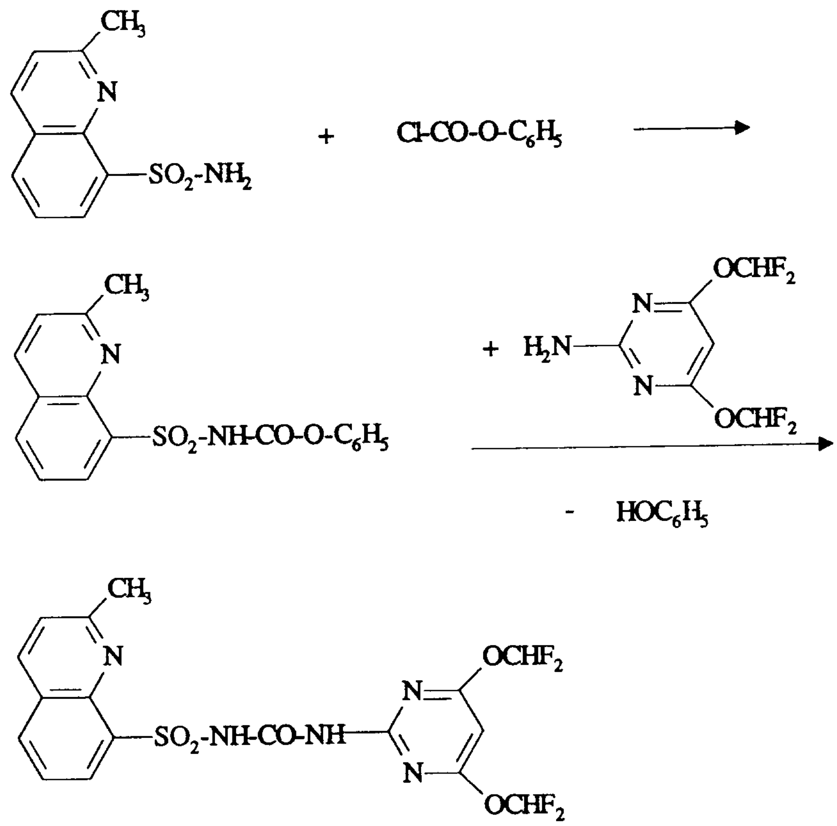


Die bei den erfindungsgemäßen Verfahren (a) und (b) zur Herstellung von Verbindungen der Formel (I) als Ausgangsstoffe zu verwendenden substituierten Chinolinsulfonsäureamide sind durch die Formel (II) allgemein definiert.

In Formel (II) hat $R^1$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^1$ angegeben wurde.

Als Beispiele für die Ausgangsstoffe der Formel (II) seien genannt:
2-Methyl-, 2-Ethyl-, 2-Propyl-, 2-Isopropyl-, 2-Butyl-, 2-Isobutyl- und 2-sec-Butylchinolin-8-sulfonsäureamid.

Die substituierten Chinolinsulfonsäureamide der Formel (II) sind noch nicht aus der Literatur bekannt und sind als neue Stoffe ebenfalls Gegenstand der vorliegenden Erfindung.

Man erhält die neuen Verbindungen der Formel (II), wenn man substituierte Clinolinsulfonsäurechloride der allgemeinen Formel (VII)

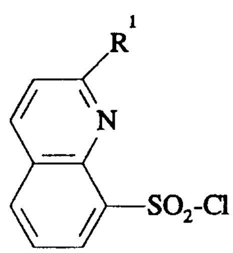
(VII)

in welcher

$R^1$ die oben angegebene Bedeutung hat,

mit Ammoniak, gegebenenfalls in Gegenwart eines Verdünnungsmittels, wie z.B. Wasser, bei Temperaturen zwischen -20 °C und +50 °C umsetzt und nach üblichen Methoden aufarbeitet (vgl. die Herstellungsbeispiele).

Die substituierten Chinolinsulfonsäurechloride der Formel (VII) sind bekannt und/oder können nach an sich bekannten Verfahren hergestellt werden (vgl. J. Serb. Chem. Soc. 52(1987), 697-704 - zitiert in Chem.

Abstracts 110:114158c; Latv. PSR Zinat. Akad. Vestis, Kim. Ser. 1975, 585 - zitiert in Chem. Abstracts 84: 59140h; Latv. PSR Zinat. Akad. Vestis, Kim. Ser. 1974,624 - zitiert in Chem. Abstracts 82: 43147p).

Man erhalt die Verbindungen der Formel (VII), wenn man substituierte Chinoline der allgemeinen Formel (VIII)

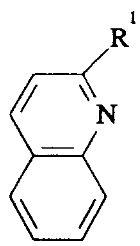

(VIII)

in welcher

$R^1$ die oben angegebene Bedeutung hat,

mit Chlorsulfonsäure und anschließend mit Thionylchlorid bei Temperaturen zwischen 0°C und 180°C umsetzt und nach üblichen Methoden aufarbeitet (vgl. die Herstellungsbeispiele).

Die beim erfindungsgemäßen Verfahren (a) zur Herstellung von Verbindungen der Formel (I) weiter als Ausgangsstoffe zu verwendenden Urethane sind durch die Formel (III) allgemein definiert

In Formel (III) haben $R^2$, X, Y und Z vorzugsweise bzw. insbesondere diejenigen Bedeutungen, die bereits oben im Zusammenhang mit der Beschreibung der erfindungsgemäß herstellbaren Verbindungen der Formel (I) vorzugsweise bzw. als insbesondere bevorzugt für $R^2$, X, Y und Z angegeben wurden;

$R^4$ steht vorzugsweise für Methyl, Ethyl, Benzyl oder Phenyl, insbesondere für Phenyl.

Als Beispiele für die Ausgangsstoffe der Formel (III) seien genannt:

N-(4,6-Dimethyl-pyrimidin-2-yl)-, N-(4-Methoxy-6-methyl-pyrimidin-2-yl)-, N-(4,6-Dimethoxy-pyrimidin-2-yl)-, N-(4-Methoxy-6-methyl-s-triazin-2-yl), N-(4-Chlor-6-methoxy-pyrimidin-2-yl)-, N-(4,6-bis-difluormethoxy-pyrimidin-2-yl)-, N-(4,6-Dimethoxy-s-triazin-2-yl)-, N-Methyl-N-(4-methoxy-6-methyl-s-triazin-2yl)- und N-Methyl-N-(4,6-dimethoxy-s-triazin-2-yl)-carbamidsäure-methylester, -ethylester, -benzylester und -phenylester.

Die Ausgangsstoffe der Formel (III) sind bekannt und/oder können nach an sich bekannten Verfahren hergestellt werden (vgl. EP-A 101670; US-P 4683000; US-P 4678500; US-P 4786311).

Man erhalt die Urethane der Formel (III), wenn man Aminoazine der allgemeinen Formel (VI)

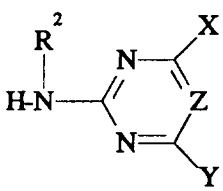

(VI)

in welcher

$R^2$, X, Y und Z die oben angegebene Bedeutung haben,

mit Chlorameisensäureestern der allgemeinen Formel (IV)

$R^4$-O-CO-Cl (IV)

in welcher

$R^4$ die oben angegebene Bedeutung hat,

gegebenenfalls in Gegenwart eines Säureakzeptors, wie z.B. Natrium(hydrogen)-carbonat, Kalium(hydrogen)carbonat, Calciumcarbonat oder Kalium-tert-butylat, und gegebenenfalls in Gegenwart eines Verdünnungsmittels, wie z.B. Cyclohexan, Methylcyclohexan, Benzol, Toluol, Xylol, Chlorbenzol, Diisopropylether, Dibutylether, Diisobutylether, Methyl-tert-burylether, Tetrahydrofuran, Dioxan, Glykoldimethylether, Aceton, Methylethylketon, Methylisobutylketon, Essigsäure-methylester, -ethylester, -propylester oder -butylester, Acetonitril, Propionitril oder Butyronitril bei Temperaturen zwischen 0°C und 150°C umsetzt (vgl. die Herstellungsbeispiele).

Die beim erfindungsgemäßen Verfahren (b) zur Herstellung von Verbindungen der Formel (I) weiterhin als Ausgangsstoffe zu verwendenden Chlorameisensäureester sind durch die Formel (IV) allgemein definiert.

In Formel (IV) steht $R^4$ vorzugsweise für Methyl, Ethyl, Benzyl oder Phenyl, insbesondere für Phenyl.

Die Ausgangsstoffe der Formel (IV) sind bekannte Synthesechemikalien.

Die in der ersten Reaktionsstufe des erfindungsgemäßen Verfahrens (b) gebildeten Chinolylsulfonylurethane der allgemeinen Formel (V) sind noch nicht aus der Literatur bekannt und sind als neue Stoffe ebenfalls Gegenstand der vorliegenden Erfindung.

Die beim erfindungsgemäßen Verfahren (b) zur Herstellung von Verbindungen der Formel (I) weiter als Ausgangsstoffe zu verwendenden Aminoazine sind durch die Formel (VI) allgemein definiert.

In Formel (VI) haben $R^2$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, X, Y und Z angegeben wurden.

Die Ausgangsstoffe der Formel (VI) sind bekannt und/oder können nach an sich bekannten Verfahren hergestellt werden (vgl. US-P 4299960; EP-A 121082; EP-A 125205; EP-A 126711; EP-A 152378; EP-A 158594).

Die erfindungsgemäßen Verfahren (a) und (b) zur Herstellung der neuen Verbindungen der Formel (I) werden vorzugsweise unter Verwendung von Verdünnungsmitteln durchgeführt Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Diburylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Säureakzeptoren können bei den erfindungsgemäßen Verfahren (a) und (b) alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetallhydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die zweite Reaktionsstufe des erfindungsgemäßen Verfahrens (b), d.h. die Umsetzung mit den Aminoazinen der Formel (VI), wird vorzugsweise in Gegenwart einer Säure durchgeführt. Als Säuren werden hierbei vorzugsweise Protonensäuren eingesetzt, wie Schwefelsäure, Methansulfonsäure, Benzolsulfonsäure und p-Toluolsulfonsäure.

Die Reaktionstemperaturen können bei den erfindungsgemäßen Verfahren (a) und (b) in einem größeren Bereich variiert werden. Im allgemeinen arbeitet man bei Temperaturen zwischen 0 °C und 100 °C, vorzugsweise bei Temperaturen zwischen 10 °C und 80 °C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine oder mehrere der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a) und (b) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen, wie z.B. in Weizen, und in dikotylen Kulturen , wie z.B. in Baumwolle, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenflache. vorzugsweise zwischen 50g und 5kg pro ha.

Die Herstellung und die Verwendung der erfindungsgemäßen Wirkstoffe geht aus den nachfolgenden Beispielen hervor.

Herstellungsbeispiele:

Beispiel 1

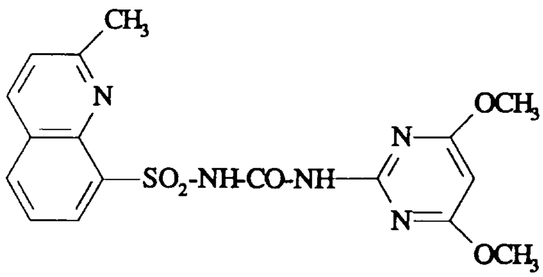

(1)

(Verfahren (a))

15,2 g (0,1 Mol) 1,8-Diazabicyclo-[5,4,0]-undec-7-en (DBU) werden bei 20 °C unter Rühren tropfenweise zu einer Mischung aus 22,7 g (0,1 Mol) 2-Methyl-chinolin-8-sulfonamid, 29,1 g (0,1 Mol) N-(4,6-Dimethoxy-pyrimidin-2-yl)carbamidsäure-phenylester und 200 ml Acetonitril gegeben. Die Reaktionsmischung wird

dann noch 30 Minuten bei 20 °C gerührt und anschließend auf ca. 1,5 Liter Eiswasser gegossen. Unter starkem Rühren werden dann langsam 11 g (0,11 Mol HCl) konz. Salzsäure zugetropft und das hierbei kristallin angefallene Produkt wird durch Absaugen isoliert

Man erhält 36 g (87% der Theorie) N-(4,6-Dimethoxy-pyrimidin-2-yl)-N'-(2-methyl-chinolin-8-yl-sulfonyl)-harnstoff vom Schmelzpunkt 199 °C.

Beispiel 2

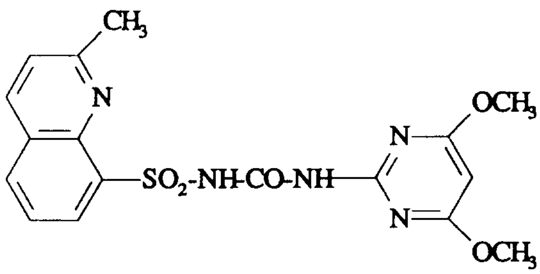

(1)

(Verfahren (b))

3,7 g (23,6 mMol) Chlorameisensäure-phenylester werden bei 15 °C bis 20 °C unter Rühren tropfenweise zu einer Mischung aus 4,5 g (19,3 mMol) 2-Methyl-chinolin-8-sulfonamid, 4,1 g (40 mMol) Triethylamin und 60 ml Acetonitril gegeben und das Reaktionsgemisch wird noch 2 Stunden bei 20 °C gerührt.

Dann werden unter Rühren 2 g (21 mMol) Methansulfonsäure und anschließend 3,1 g (20 mMol) 2-Amino-4,6-dimethoxy-pyrimidin dazu gegeben und die Reaktionsmischung wird dann 6 Stunden bei 60 °C gerührt. Nach weiteren 12 Stunden Rühren bei 20 °C wird auf 0 °C abgekühlt und dann das kristallin angefallene Produkt durch Absaugen isoliert.

Man erhält 5 g (61% der Theorie) N-(4,6-Dimethoxy-pyrimidin-2-yl)-N'-(2-methylchinolin-8-yl-sulfonyl)-harnstoff vom Schmelzpunkt 199 °C.

Analog zu den Beispielen 1 und 2 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

Tabelle 2: Herstellungsbeispiele für die Verbindungen der Formel (I)

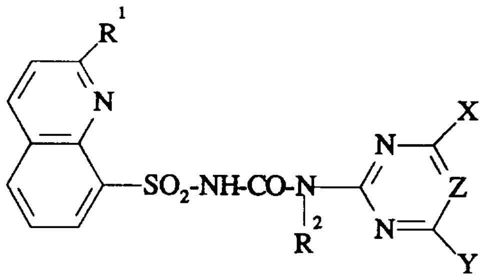

(I)

| Bsp.-Nr. | $R^1$ | $R^2$ | X | Y | Z | Schmelzpunkt (°C) |
|---|---|---|---|---|---|---|
| 3 | $CH_3$ | $CH_3$ | $OCH_3$ | $OCH_3$ | N | 205 * |
| 4 | $CH_3$ | $CH_3$ | $CH_3$ | $OCH_3$ | N | 215 * |
| 5 | $CH_3$ | H | $CF_3$ | $OCHF_2$ | CH | 148 |
| 6 | $CH_3$ | H | $CH_3$ | $OCH_3$ | CH | 182 |
| 7 | $CH_3$ | H | $CH_3$ | $CH_3$ | CH | 224 |
| 8 | $CH_3$ | H | Cl | $OCH_3$ | CH | 182 |
| 9 | $CH_3$ | H | $OCHF_2$ | $OCHF_2$ | CH | 124 |
| 10 | $C_2H_5$ | H | $OCH_3$ | $OCH_3$ | CH | 185 |
| 11 | $i-C_4H_9$ | H | $OCH_3$ | $OCH_3$ | CH | 201 |

* erhalten als Natrium-Salz

Ausgangsstoffe der Formel (II):

Beispiel (II-1)

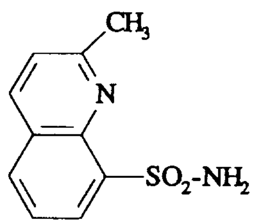

13,5 g (52 mMol) 2-Methyl-chinolin-8-sulfonsäurechlorid werden bei 20 °C zu 100 ml einer 25%igen wäßrigen Ammoniaklösung gegeben und die Mischung wird 2 Stunden kräftig gerührt. Dann wird das kristallin angefallene Produkt durch Absaugen isoliert.

Man erhält 11,3 g (95% der Theorie) 2-Methyl-chinolin-8-sulfonamid vom Schmelzpunkt 174 °C.

Analog Beispiel (II-1) können beispielsweise auch die in der nachstehenden Tabelle 3 aufgeführten Verbindungen der Formel (II) hergestellt werden:

Tabelle 3: Beispiele für die Verbindungen der Formel (II)

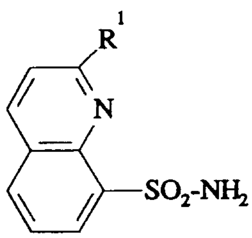

(II)

| Bsp.-Nr. | $R^1$ | Schmelzpunkt (°C) |
|---|---|---|
| II-2 | $C_2H_5$ | 138 |
| II-3 | $n\text{-}C_3H_7$ | |
| II-4 | $i\text{-}C_3H_7$ | |
| II-5 | $n\text{-}C_4H_9$ | |
| II-6 | $i\text{-}C_4H_9$ | |
| II-7 | $s\text{-}C_4H_9$ | |

Ausgangsstoffe der Formel (III):

Beispiel (III-1)

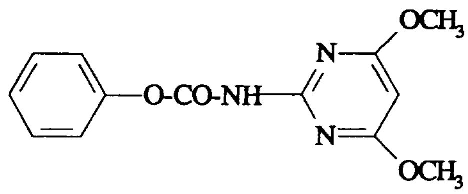

172 g (1,1 Mol) Chlorameisensäure-phenylester werden bei 20 ° C unter Rühren tropfenweise zu einer Mischung aus 155 g (1,0 Mol) 2-Amino-4,6-dimethoxypyrimidin, 200 g (2,0 Mol) Calciumcarbonat (Pulver) und 1,5 Liter Tetrahydrofuran gegeben und das Reaktionsgemisch wird 12 Stunden bei 50 ° C gerührt. Dann wird abgesaugt, mit Tetrahydrofuran nachgewaschen, das Filtrat im Wasserstrahlvakuum eingeengt und der verbleibende Rückstand durch Verreiben mit Diethylether zur Kristallisation gebracht.

Man erhält 155 g (53% der Theorie) N-(4,6-Dimethoxy-pyrimidin-2-yl)-carbamidsäure-phenylester vom Schmelzpunkt 118 ° C.

Ausgangsstoffe der Formel (VII):

Beispiel (VII-1)

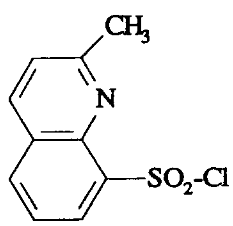

143 g (1,0 Mol) 2-Methyl-chinolin (Chinaldin) werden im Verlauf von 30 Minuten tropfenweise zu 500 g (4,3 Mol) Chlorsulfonsäure gegeben, wobei die Innentemperatur auf ca. 110 ° C ansteigt. Das Gemisch wird dann 3 Stunden bei 150 ° C gerührt und nach Abkühlen auf Raumtemperatur (ca. 20 ° C) werden 238 g (2,0 Mol) Thionylchlorid tropfenweise dazu gegeben. Nach Abklingen der Gasentwicklung wird das Gemisch noch eine Stunde unter Rückfluß erhitzt, dann auf Raumtemperatur abgekühlt und tropfenweise zu einer Mischung aus ca. 3 Liter Eiswasser und 1,5 Liter Essigsäureethylester unter Rühren gegeben. Die organische Phase wird dann abgetrennt, die wäßrige Phase mit 500 ml Essigsäureethylester nachextrahiert, die vereinigten organischen Phasen mit Wasser gewaschen, mit Natriumsulfat getrocknet und filtriert. Das Filtrat wird eingeengt, wobei das Produkt als kristalliner Rückstand erhalten wird.

Ausbeute: 142 g (58% der Theorie)
Schmelzpunkt: 113 ° C.

Anwendungsbeispiele:

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Herstellungsbeispielen 1 und 6 bei guter Verträglichkeit gegenüber Baumwolle starke Wirkung gegen Unkräuter.

Bei einer Aufwandmenge von 125 g/ha zeigen diese Verbindungen gegen die Unkräuter Alopecurus, Chenopodium, Galium, Galinsoga und Matricaria eine Wirkung (Schädigung) von 80 bis 95 %, ohne die Baumwollpflanzen zu schädigen.

Beispiel B

Post-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

In diesem Test zeigt beispielsweise die Verbindung gemäß Herstellungsbeispiel 1 bei guter Verträglichkeit gegenüber Weizen starke Wirkung gegen Unkräuter.

Bei einer Aufwandmenge von 125 g/ha zeigt diese Verbindung gegen die Unkräuter Digitaria und Sorghum eine Wirkung (Schädigung) von 95 % und gegen die Unkräuter Abutilon, Cassia, Datura, Galinsoga, Sinapis und Xanthium von jeweils 100 %, ohne die Weizenpflanzen zu schädigen.

**Patentansprüche**

**1.** Substituierte Chinolylsulfonylharnstoffe der allgemeinen Formel (I)

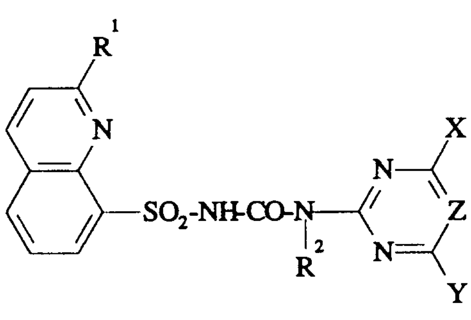

(I)

in welcher

$R^1$ für gegebenenfalls durch Halogen substituiertes $C_1$-$C_6$-Alkyl steht,

$R^2$ für Wasserstoff oder für gegebenenfalls durch Halogen, Cyano, Carboxy, $C_1$-$C_4$-Alkoxy oder $C_1$-$C_4$-Alkoxy-carbonyl substituiertes $C_1$-$C_6$-Alkyl, für jeweils gegebenenfalls durch Halogen substituiertes $C_3$-$C_6$-Alkenyl oder $C_3$-$C_6$-Alkinyl, oder für gegebenenfalls durch Halogen, Cyano, $C_1$-$C_4$-Alkyl, $C_1$-$C_4$-Alkoxy oder $C_1$-$C_4$-Alkoxy-carbonyl substituiertes Phenyl-$C_1$-$C_2$-

Alkyl steht,

X für Wasserstoff, Halogen, Cyclopropyl oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_3$-alkyl)-amino steht,

Y für Wasserstoff, Halogen oder für jeweils gegebenenfalls durch Halogen oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylamino oder Di-($C_1$-$C_3$-alkyl)-amino steht und

Z für Stickstoff oder die Gruppierung C-$R^3$ steht,

worin

$R^3$ für Wasserstoff, Halogen, $C_1$-$C_4$-Alkyl oder $C_1$-$C_4$-Alkoxy steht,

sowie Salze von Verbindungen der Formel (I), wobei die Verbindung N-(4,6-Dimethyl-s-triazin-2-yl)-N'-(2-methyl-chinolin-8-yl-sulfonyl)-harnstoff ausgenommen ist.

**2.** Verbindungen der Formel (I) und deren Salze gemäß Anspruch 1, dadurch gekennzeichnet, daß darin

$R^1$ für gegebenenfalls durch Fluor und/oder Chlor substituiertes $C_1$-$C_4$-Alkyl steht,

$R^2$ für Wasserstoff oder für gegebenenfalls durch Fluor, Chlor, Cyano, Carboxy, Methoxy, Ethoxy, Methoxycarbonyl oder Ethoxycarbonyl substituiertes $C_1$-$C_4$-Alkyl, für jeweils gegebenenfalls durch Fluor, Chlor und/oder Brom substituiertes $C_3$-$C_4$-Alkenyl oder $C_3$-$C_4$-Alkinyl steht, oder für gegebenenfalls durch Fluor, Chlor, Brom, Cyano, Methyl, Ethyl, Methoxy, Ethoxy, Methoxycarbonyl oder Ethoxycarbonyl substituiertes Benzyl steht,

X für Wasserstoff, Fluor, Chlor, Brom, Cyclopropyl oder für jeweils gegebenenfalls durch Fluor, Chlor, Methoxy oder Ethoxy substituiertes $C_1$-$C_3$-Alkyl, $C_1$-$C_3$-Alkoxy, $C_1$-$C_3$-Alkylthio, $C_1$-$C_3$-Alkylamino oder Di-($C_1$-$C_2$-alkyl)-amino steht,

Y für Wasserstoff, Fluor, Chlor, Brom, oder für jeweils gegebenenfalls durch Fluor, Chlor, Methoxy oder Ethoxy substituiertes $C_1$-$C_3$-Alkyl, $C_1$-$C_3$-Alkoxy, $C_1$-$C_3$-Alkylthio, $C_1$-$C_3$-Alkylamino oder Di-($C_1$-$C_2$-alkyl)-amino steht und

Z für Stickstoff oder die Gruppierung C-$R^3$ steht, worin

$R^3$ für Wasserstoff, Fluor, Chlor, Brom, Methyl, Ethyl, Methoxy oder Ethoxy steht,

ausgenommen die Verbindung N-(4,6-Dimethyl-s-triazin-2-yl)-N'-(2-methyl-chinolin-8-yl-sulfonyl)-harnstoff.

**3.** Verbindungen der Formel (I) und deren Salze gemäß Anspruch 1, dadurch gekennzeichnet, daß darin

$R^1$ für Methyl, Ethyl, Propyl oder Isopropyl steht,

$R^2$ für Wasserstoff oder Methyl steht,

X für Wasserstoff, Chlor, Cyclopropyl, Methyl, Ethyl, Trifluormethyl, Methoxymethyl, Methoxy, Ethoxy, Difluormethoxy, Methylthio, Ethylthio, Methylamino, Ethylamino oder Dimethylamino steht,

Y für Wasserstoff, Chlor, Trifluormethyl, Methoxy, Ethoxy, Difluormethoxy, Methylthio, Ethylthio, Methylamino, Ethylamino oder Dimethylamino steht und

Z für Stickstoff oder die Gruppierung C-$R^3$ steht, worin

$R^3$ für Wasserstoff steht.

**4.** Verfahren zur Herstellung von substituierten Chinolylsulfonylharnstoffe der allgemeinen Formel (I) und deren Salzen gemäß Anspruch 1, dadurch gekennzeichnet, daß man

(a) substituierte Chinolinsulfonsäureamide der allgemeinen Formel (II)

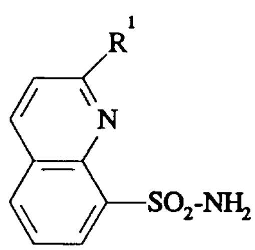

(II)

in welcher

$R^1$ die in Anspruch 1 angegebene Bedeutung hat,

mit Urethanen (Carbamaten) der allgemeinen Formel (III),

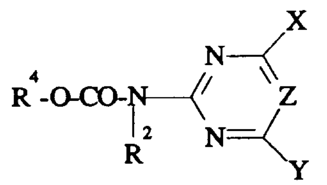 (III)

in welcher
$R^2$, X, Y und Z die in Anspruch 1 angegebene Bedeutung haben und
$R^4$ für $C_1$-$C_4$-Alkyl, Benzyl oder Phenyl steht,
gegebenenfalls in Gegenwart eines Säureakzeptors und gegebenenfalls in Gegenwart eines Verdünnungsmittels umsetzt, oder wenn man
(b) substituierte Chinolinsulfonsäureamide der allgemeinen Formel (II),

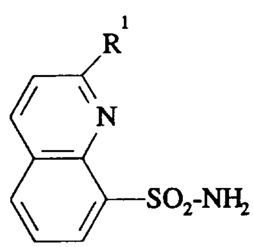 (II)

in welcher
$R^1$ die in Anspruch 1 angegebene Bedeutung hat,
in einer ersten Reaktionsstufe mit Chlorameisensäureestern der allgemeinen Formel (IV),

$Cl-CO-O-R^4$ (IV)

in welcher
$R^4$ die in Anspruch 1 angegebene Bedeutung hat,
gegebenenfalls in Gegenwart eines Säureakzeptors und gegebenenfalls in Gegenwart eines Verdünnungsmittels umsetzt, und die hierbei gebildeten Chinolylsulfonylurethane der allgemeinen Formel (V),

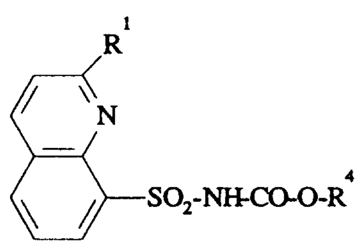 (V)

in welcher
$R^1$ und $R^4$ die in Anspruch 1 angegebene Bedeutung haben,
in einer zweiten Reaktionsstufe mit Aminoazinen der allgemeinen Formel (VI),

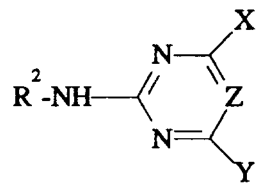

(VI)

in welcher
$R^2$, X, Y und Z die in Anspruch 1 angegebene Bedeutung haben,
gegebenenfalls nach Zugabe einer Säure umsetzt,
und gegebenenfalls die nach Verfahren (a) oder (b) erhaltenen substituierten Chinolylsulfonylharnstoffe nach üblichen Methoden in Salze überführt.

**5.** Herbizide Mittel, gekennzeichnet durch einen Gehalt an mindestens einer Verbindung der Formel (1) gemäß Anspruch 1.

**6.** Verwendung von Verbindungen der allgemeinen Formel (I) gemäß Anspruch 1 zur Bekämpfung von unerwünschtem Pflanzenwachstum.

**7.** Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

**8.** Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

**9.** Substituierte Chinolinsulfonsäureamide der allgemeinen Formel (II),

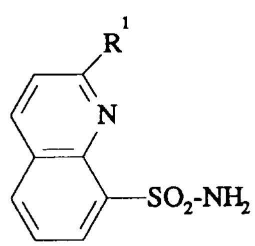

(II)

in welcher
$R^1$ für gegebenenfalls durch Halogen substituiertes $C_1$-$C_6$-Alkyl steht.

**10.** Chinolylsulfonylurethane der allgemeinen Formel (V),

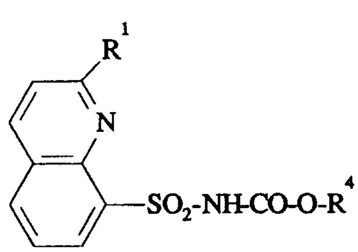

(V)

in welcher
$R^1$ für gegebenenfalls durch Halogen substituiertes $C_1$-$C_6$-Alkyl steht und
$R^4$ für $C_1$-$C_4$-Alkyl, Benzyl oder Phenyl steht.

Europäisches Patentamt

# EUROPÄISCHER RECHERCHENBERICHT

Nummer der Anmeldung
EP 93 11 8990

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | EP-A-0 051 465 (E.I. DU PONT DE NEMOURS AND COMPANY) 12. Mai 1982<br>* gesamtes Dokument; insbesondere Verbindungen der Formeln I, II *<br>--- | 1-10 | C07D401/12<br>A01N47/36 |
| A | CHEMICAL ABSTRACTS, vol. 92, no. 13, 31. März 1980, Columbus, Ohio, US; abstract no. 110635t, F. MONTORO ET AL. 'Synthesis and pharmacological properties of new oral antidiabetic drugs'<br>Seite 626 ;<br>* Zusammenfassung *<br>& Congr. Nac. Farm. Ind., (Actas), 1st 1977 (Pub. 1978) 320-6<br>--- | 1-4,9,10 | |
| A | CHEMICAL ABSTRACTS, vol. 117, no. 9, 31. August 1992, Columbus, Ohio, US; abstract no. 90251u, K. NAGARAJAN ET AL. 'Condensed heterotricyclics: synthesis of 7H-8,9-dihydropyrido(3,2,1-ij)(1,2,4)benzothiadiazine 1,1-dioxide derivatives'<br>Seite 779 ;<br>* Zusammenfassung *<br>& Indian J. Chem., Sect. B 31B (6) 310-15 (1992)<br>--- | 1-3 | RECHERCHIERTE SACHGEBIETE (Int.Cl.5)<br>C07D<br>A01N |
| A | CHEMICAL ABSTRACTS, vol. 93, no. 7, 18. August 1980, Columbus, Ohio, US; abstract no. 71572u, F. MONTORO ET AL. '8-Quinolinesulfonylureas'<br>Seite 959 ;<br>* Zusammenfassung *<br>& ES-A-479097 (1980)<br>----- | 1-4,9,10 | |

Der vorliegende Recherchenbericht wurde für alle Patentansprüche erstellt

| Recherchenort | Abschlußdatum der Recherche | Prüfer |
|---|---|---|
| MÜNCHEN | 15. April 1994 | Herz, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)